# EUROPÄISCHE PATENTANMELDUNG

(11) **EP 1 987 812 A1**
(43) Veröffentlichungstag der Anmeldung: **05.11.2008**
(21) Anmeldenummer: 07008201.1
(22) Anmeldetag: 23.04.2007
(51) Int. Cl.: A61K 8/73, A61Q 19/06

(54) **Tagesrhytmisch angepasste Anwendung von Hyaferm bei Cellulite**

(71) Anmelder: Fibona Health Products GmbH, 65232 Taunusstein (DE)
(72) Erfinder: Arlt, Stefan-Alexander, 65193 Wiesbaden (DE); Smoljo, Seka, 65193 Wiesbaden (DE); Gerber, Gerhard Professor Dr., 10319 Berlin (DE); Westphal, Günter Prof. Dr., 10319 Berlin (DE)
(74) Vertreter: Sammer, Dietrich

(57) **Zusammenfassung**

Die Erfindung betrifft ein Produkt, das den Tages- und Nachtrhytmus der Haut beachtet und benutzt, um Auslagerung zu beschleunigen (Tag) und Einlagerungen zu verhindern (Nacht) sowie ein Verfahren zur Sicherung der effizienten Permeation von Wirkstoffen bei Cellulite unter Zusatz von Hyaferm(R).

## Beschreibung

### 1. Darstellung der Erfindung

Tagesrhythmisch angepasste Anwendung von Hyaferm^{®} bei Cellulite

Die Erfindung betrifft ein Produkt, das den Tages- und Nachtrhythmus der Haut- besonders des Unterhautfettgewebes - beachtet und benutzt, um Auslagerungen zu beschleunigen (Tag) und Einlagerungen zu verhindern (Nacht).

### a) Einschlägiger Stand der Forschung mit Fundstelle

### a.1)Biologische Grundlagen unserer "inneren Uhr"

Die Periodizität ist eine fundamentale Eigenschaft biologischer Abläufe. Die augenfälligste Form ist das Schlaf-Wach-Verhalten des erwachsenen Menschen, das eine zirka 24-Stunden-Rhythmik aufweist. Inzwischen ist anerkannt, dass in allen Zellen und Organismen periodische Schwankungen der verschiedenen biologischen Funktionen auftreten. Damit beschäftigt sich die Chronobiologie. Das trifft für die einfachsten Bakterien, für einzellige Lebewesen, für Pflanzen, Tiere und den Menschen zu. In Blutzellen, die dem Körper entnommen werden, setzt sich das periodische Verhalten fort. Teilt sich eine Zelle, dann setzt sich die Rhythmik in den Tochterzellen fort.

War man anhand des Schlaf-Wach-Rhythmus beim Menschen und der tageszeitabhängigen Veränderungen bei Pflanzen (Öffnen von Blüten) zunächst der Auffassung, dass exogene Faktoren (HellDunkel-Phasen) die Ursache dieser zirkadianen (24 h) Phänomene sind, so weiß man heute, dass diesen ein komplexes inneres Gefüge verschiedener Zeitgeber (Oszillatoren) zugrunde liegt. Molekularbiologische Analysen erbrachten den Nachweis positiver und negativer regulativer Elemente im Genom. Sie konstituieren eine zellautonome Rückkopplungsschleife, in die Transkriptions-(Ablesemechanismen der genetischen Information) und Translationsmechanismen (Eiweißsynthese) eingeschlossen sind. Die aktivierenden Elemente treiben das System an und gleichzeitig werden auch die hemmenden angeschaltet, die im Zuge der Rückkopplung die positiven Elemente wieder zügeln. Durch das Synchronisieren mehrerer molekularer Oszillatoren gibt sich die Zelle eine "innere Uhr", die viele Zellfunktionen wie Proteinsynthese, Zellteilungszyklus, Stoffwechsel usw. beeinflusst. Inzwischen kennt man acht "Uhrengene" (Reppert und Weaver 2001). Neueste Forschungsergebnisse weisen auf eine Rolle dieser "Uhrenmoleküle" als Stresssensoren (Empfänger von Stresssignalen) hin (Ishida 2007). Eine Vorstellung von der Reichweite der Wirkungen dieser Uhrengene geben Zvonic et al. (2006), die in Fettgewebe und Leber eine zirkadiane Rhythmik in der Expression (Synthese der entsprechenden Eiweiße) von 650 Genen nachwiesen.

Mit molekulargenetischen Methoden ist es jetzt möglich, einzelne Bereiche des Genoms abzuschalten. Mäuse, denen eines der "Uhrengene" stillgelegt wird, zeigen eine gestörte Tag-/Nacht-Aktivität, sie fressen mehr, altem vorzeitig und haben eine verkürzte Lebenserwartung, einen gestörten Stoffwechsel des Unterhautfettgewebes, geschrumpfte Organe und in einigen Geweben eine erhöhte Belastung mit Sauerstoffradikalen. Diese Veränderungen werden mit unangepassten Reaktionen auf Stress in Verbindung gebracht (Kondratov et al. 2006, Vanitallie et al. 2006). Untersuchungsergebnisse von Shimba et al. (2005) weisen auf eine fördernde Rolle von Uhrengenen auf die Differenzierung von Bindegewebszellen (Fibroblasten) in Fettzellen hin. Demnach beeinflussen molekulare Taktgeber nicht nur Einbau und Mobilisation der Fette und damit den Füllungszustand der Fettzellen, sondern auch die Anzahl dieser Zellen.

Die molekularen und biochemischen Uhren der einzelnen Zellen und Gewebe unseres Körpers können zwar eigenständig "ticken", aber sie können sich aus sich heraus nicht zu einer inneren Uhr des Gesamtorganismus konstituieren. Diese Aufgabe obliegt einem Kontrollsystem in bestimmten Neuronengruppen (Neuron = Himzelle) im Hypothalamus.- eines zum Zwischenhirn gehörenden Himbereichs. Zwei äußere Stellgrößen sind von besonderer Bedeutung für die Taktung dieser Zentraluhr: der Hell-Dunkel-Wechsel zwischen Tag und Nacht und der Füllungsgrad der Zellen des Fettgewebes.

Neuronengruppen im Nucleus suprachiasmaticus (NSC) des Hypothalamus reagieren auf den exogenen Zeitgeber Licht. In der Retina des Auges befinden sich nicht nur die klassischen Photorezeptoren zum Schwarz-Weiß- und Farbensehen, sondern auch spezielle lichtempfindliche Ganglienzellen mit direkter Verbindung zum Gehirn. Lichtsignale, die von diesen Ganglienzellen registriert werden, gelangen über den Tractus retinohypothalamicus direkt zum NSC und synchronisieren die einzelnen neuronalen Oszillatoren. Diese Zellen werden damit hinsichtlich elektrische Aktivität, Glucoseverbrauch, Hormonproduktion (GABA, Vasopressin, Glutaminsäure) und Proteinsynthese auf eine 24-stündige Periodik im Einklang mit dem Tag-Nacht-Zyklus eingestellt.

In deren Nachbarschaft gelegene Nervenzellgruppen erhalten aus den Fettgeweben Informationen über den Einbau und die Mobilisation von Fett und über den Füllungszustand der Fettzellen. Der Bote dieser Informationen ist ein Proteohormon - das Leptin. Dabei ist zwischen kurzfristiger Kontrolle (Wechsel zwischen Sättigung und Hunger) und längerfristigen Verschiebungen der Körperfettmasse zu unterscheiden (Zunahme bzw. Abnahme des Körpergewichtes). Leptin wird von den Fettzellen in Abhängigkeit vom Füllungszustand und der Aktivität des Fetteinbaus bzw. der Fettmobilisation abgesondert. Mit dem Blut gelangt es in das Gehirn.

NSC und die benachbarten Himareale verarbeiten die Signale Hell/Dunkel und Zustand des Fettgewebes, koordinieren die Funktionen anderer Himbereiche (Schlaf-Wachrhythmus, Hungergefühl) und der peripheren Gewebe (Strubbe und van Dijk 2002). Der letztgenannten Funktion dient die Signalkaskade Hypothalamus→Hypophyse→Nebennierenrinde. Angeregt durch die NSC-Neurone gibt der Hypothalamus CRF (Corticotropin releasing factor) an die Hypophyse (Himanhangsdrüse) ab und diese wird dadurch zur Freisetzung von ACTH aktiviert (Adrenocorticotrophic hormone), Das Hormon gelangt über das Blut in die Nebenniere und löst die Bildung von Glucocorticoiden (u. a. Cortisol) aus. Diese haben multiple Funktionen auf alle Gewebe unseres Körpers, u. a. steigern sie die Leptinsynthese (Wabitsch et al. 1996, Larsson und Ahren 1996, Miell et al, 1996). Ausdruck dieser Beziehung ist die beim Menschen von Elimam et al. (1998) unter Basalbedingungen ermittelte Korrelation der Blutplasmawerte von Cortisol und Leptin. Offensichtlich ist der im Hypothalamus erfolgende Abgleich zwischen der Tagesrhythmik und der homöostatischen Kontrolle der Fettmasse von grundsätzlicher Bedeutung. Im Zusammenspiel mit Sättigungssignalen aus dem Magen-Darm-Bereich werden Häufigkeit und Menge der Nahrungsaufnahme, Schlaf-Wach-Periodik und andere Verhaltensweisen festgelegt.

Die relative Konstanz der Fettmasse und der Tag-Nacht-Rhythmik geben dem Gefüge der signalgebenden Elemente eine hohe Stabilität. Das Erlebnis des "jet-lag" ist ein bekanntes Beispiel. Der menschliche Körper benötigt etwa einen Tag um sich an eine Zeitdifferenz von einer Stunde anzupassen, d. h. also eine Woche bei einer Zeitdifferenz von sieben Stunden. Ebenso ist eine nachhaltige Verringerung der Körperfettmasse nur schwer zu erreichen ("re-baund effect" nach Fastenkuren).

in der Medizin erlangt die Tagesrhythmik große Aufmerksamkeit im Hinblick auf die Wirkungen und Nebenwirkungen (!) von Arzneimitteln. Dass die Beeinflussung periodisch wechselnder physiologischer Funktionen durch ein Medikament von der Phase des zirkadianen Rhythmus abhängt, zu dem es gegeben wird, ist leicht einzusehen. Aber auch Veränderungen in Aufnahme und Abbau eines Medikaments lassen sich auf der Grundlage biologischer Rhythmen erklären. Chronopharmakologie heißt die wissenschaftliche Fachrichtung, die die Zeitabhängigkeit der Wirkungen und Nebenwirkungen von Pharmaka in Relation zu biologischen Rhythmen untersucht.

### a.2)Tagesrhythmik des Fettgewebes und der Haut

Forschungsergebnisse der letzten Jahre lösten ein grundsätzliches Umdenken in unserem Verständnis zum Fettgewebe aus. Sah man vordem dieses Gewebe lediglich als Speicher für im Überschuss aufgenommene Nährstoffe an, so ist es jetzt als äußerst dynamisch akzeptiert, eingebunden in biochemische, physiologische, Immun- und endokrine Funktionen. Ein wechselseitiger Stoff- und Informafionsaustausch existiert mit der Immunabwehr, der Hypothatamus-Hypophyse-Nebennieren-Achse, dem sympathischen Nervensystem, dem Blutkreislaufsystem, der Blutgerinnung, dem Gleichgewicht unter den Sexualhormonen und verschiedenen trophischen Funktionen. Es ist deshalb folgerichtig zu vermuten, dass tagesrhythmische Muster auch für dieses Gewebe gelten.

Der Vielfalt unter den Funktionen des Fettgewebes liegt die variable Zusammensetzung aus unterschiedlichen Zelltypen zugrunde. Nur etwa die Hälfte seiner Zellen sind typische Fettgewebszellen (Adipocyten), der Rest sind Bindegewebs- und Immunzellen sowie verschiedene Zellen der Blutgefäße (Endothelzellen). Sie unterliegen eigenen inneren Periodizitäten und werden durch die zentrale Schaltuhr des Gehirns getaktet.

Damit folgt das Fettgewebe auch der Tag-Nacht-Rhythmik. Sie ist besonders in jenem stoffwechselaktiveren intestinalen Fett ausgeprägt, das die Organe des Bauchraumes umgibt (Darm, Nieren, Bauchspeicheldrüse). Prinzipiell trifft das auch auf das Unterhautfettgewebe zu, d. h. jenes Fett im Hüft- und Oberschenkeibereich, das für die Ausprägung der Cellulite verantwortlich ist-wenn auch in geringerem Grade.

In dem Kontrollsystem zur Konstanthaltung der Fettmasse (Energiehomöostase) spielen Leptin und Insulin eine entscheidende Rolle (Havel et al. 1996). Beide informieren den Hypothalamus über den Zustand der Fettgewebe und lösen Wirkungen in zwei Richtungen aus:
• Unterdrückung der Signalwege, die eine Erhöhung der Nahrungsaufnahme und Zunahme der Fettmasse bewirken
• Aktivierung von Signalwegen, die den Katabolismus (Abbau) von Fett veranlassen.

Neben der Funktion als Signalgeber für das Gehirn übt Leptin noch eine unmittelbare Wirkung auf die Fettzelle aus (Zhou et al. 1998). Es induziert das Entkopplungsprotein, das durch die Entkopplung der mitochondrialen ATP-Produktion vom Sauerstoffverbrauch den Abbau des gespeicherten Fettes und die Oxydation der freigesetzten Fettsäuren unter Wärmebildung in Gang setzt.

Folgende Veränderungen verschieben das Gleichgewicht Fetteinbau/Fettabbau in Richtung Einbau: Alterung, Übergewicht, Alkoholexzess, Rauchen, Depressionen, Schlafstörungen, einige Erkrankungen (Morbus Cushing, Diabetes, polycystisches ovarielles Syndrom) (Bray und Young 2007). Das Wachstumshormon und Sexualhormone (Östrogene, Testosteron) aktivieren den Fettabbau (Bjomtorp 1996,1997). Die Wirksamkeit dieser Hormone nimmt im Alter und bei den genannten Erkrankungen und Beispielen ab, was für die Fettmassezunahme in den höheren Lebensabschnitten verantwortlich gemacht wird.

Jedes geregelte System kann nur funktionieren, wenn seine Komponenten nicht starr festgelegt sind, sondern in gewissen Grenzen pulsieren (oszillieren). Die für den Einbau und für die Mobilisierung von Fett verantwortlichen Hormone unterliegen einer mehr oder weniger ausgeprägten Tagesrhythmik mit pulsatiler Modulation. Leptin und Insulin zeigen morgens die niedrigsten Werte im Blut, sie nehmen im Verlaufe des Tages zu und erreichen um Mitternacht Gipfelwerte (Gasquet de et al. 1977, Wu et al. 1986, Matkovic et al. 1997, Saad et al. 1998, Simon et al. 1998, Coleman und Herrmann 1999, Wolthers et al, 1999, Ankarberg-Lindgren et al. 2001). Die Cortisolkonzentrationen im Blut verhalten sich anders. Sie durchlaufen das Minimum mitternachts, nehmen bis zum Morgen zu, erreichen am späten Vormittag das Maximum und fallen danach wieder ab. In der Summe führen diese Verläufe tagsüber zu einer Fettmobilisation und in der Nacht zum Fetteinbau. In Übereinstimmung mit dieser Rhythmik ist die der LPL (Lipoproteinlipase), des für den Einbau verantwortlichen Enzyms. Seine Aktivität ist am höchsten in der Nacht und am frühen Morgen und durchläuft ein Minimum am Nachmittag (Bergo et al. 1996, Asaradnam et al. 2002, ). Wirkstoffe mit beta-adrenerger Wirkung vermindern die Aktivität dieses Enzyms und fördern den Fettabbau. Dazu gehören Koffein und Yohimbin (McCarthy 2001). Die Dynamik des Fettgewebestoffwechsets widerspiegelt sich unter anderem in der Körpertemperatur. Der tagsüber ablaufende Fettabbau bewirkt eine Erwärmung um etwa 0,6 Grad. Die Abnahme der Schwankungsbreite der Leptinoszillationen im fortgeschrittenen Alter spricht für eine gleitende Verschiebung der Aktivität der Signalgeber und -empfänger (Franceschini et al. 1999). Zeitweilige Hungerphasen (Fastenkuren) führen zu einer Phasenverschiebung in der Expression der "Uhrengene" und ihrer Signalempfänger im Fettgewebe (Zvonic et al. 2006).

Einige Ergebnisse machen auf das tagesrhythmische Verhalten anderer Bestandteile des Fettgewebes und der Haut aufmerksam. Anhand von Blutuntersuchungen von Kollagenfragmenten - des mengenmäßig größten Bestandteils des Hautbindegewebes - lassen sich Aussagen zur Synthese und zum Abbau dieses Struktureiweißes machen. Bei Frauen im Alter von 32 ± 5 Jahren wurde eine Tagesrhythmik mit einem Maximum in der Nacht und einem Minimum am Nachmittag festgestellt (Hassager et al. 1992), Bei zweijährigen Stuten fanden Jackson et al. (2003) die höchsten Einbauwerte in den frühen Morgenstunden.

Neueste Daten von Hayashi (2007) unterstreichen eine äußerst stabile molekulare innere Uhr des angeborenen Immunsystems. Die "Uhrengene" der Makrophagen gleichen denen im Hypothalamus. Phagocytose und die Synthese von Cytokinen und Chemokinen (Signalstoffe der Immunabwehr) durchlaufen ein Minimum in der Nacht und besitzen tagsüber die höchsten Aktivitäten. Dieser Zyklus ist nicht an die Anwesenheit von Bakterien gebunden.

### b) Würdigung des Standes der Forschung

Eine gründliche Recherche in den Datenbanken ergibt keinen Hinweis auf Untersuchungen zur Tag-Nacht-Rhythmik von Cellulite, auch nicht zu Veränderungen von Faktoren, die auf das Fett- und Bindegewebe der Haut bei Cellulite einwirken, wie Insulin, Leptin, Cortisol usw. Trotzdem geht man vermutlich nicht fehl, wenn man die an anderen Bereichen des Fett- und Bindegewebes gewonnenen Erkenntnisse auf die Haut unter den Bedingungen der Veränderungen bei Cellulite überträgt und eine Tagesrhythmik postuliert.

Zur Erklärung der Cellulite werden im wesentlichen folgende vier Haupthypothesen diskutiert:
• Unterschiedliche Gewebearchitektur der Haut bei Frauen und Männern
• Veränderungen der Bindegewebssepten
• Veränderungen im Blutgefäßsystem
• Entzündungsfaktoren.

Das Verhältnis der Anteile zwischen Fettgewebe und Bindegewebe in einem gegebenen Volumen der Unterhaut korreliert mit dem Schweregrad der Cellulite (Mirrashed et al. 2004). Den diskutierten Annahmen und Erklärungsversuchen zur Cellulite ist gemeinsam, dass die neuen Erkenntnisse über das Cytokinnetzwerk der Haut und über das Fettgewebe als endokrines Organ nicht berücksichtigt werden. Der hohe Organisationsgrad der Haut widerspiegelt sich nicht nur in einem regen Stoffwechsel, in der rapiden Erneuerung seiner Proteinstrukturen und im regen Signalaustausch untereinander und mit anderen Geweben einschließlich des Gehirns, sondern auch in einer retativ stabilen Tag-Nacht-Rhythmik seiner physiologischen Abläufe.

Als logische Konsequenz folgt die Weiterführung des Glucaferm^{®}/Hyaferm^{®}-Konzeptes der Fibona Health Products GmbH Taunusstein. Anhand der zirkadianen Rhythmik von Leptin, Insulin, Cortisol, Lipoproteinlipase und der Körpertemperatur kann von einer Phase des Fetteinbaus in das Unterhautfettgewebe in der Nacht und der Mobilisation gespeicherten Fettes am Tage ausgegangen werden. Vom Hyaferm^{®} ist die fördernde Wirkung auf die Permeation auch höhermolekularer Wirkstoffe in die Haut bekannt (Meyer et al. 2006).

Durch Einmischung eines geeigneten(r) Wirkstoffe(s) soll die Tages-Nacht-Rhythmik der Cellulite-Haut auf zweifache Weise beeinflusst werden:
• Schwächung des Fetteinbaus in der Nachtphase
• Förderung der Fettmobilisation in der Tagphase.

Durch den Hyaferm^{®}-Zusatz soll die Permeation der/des Wirkstoffe(s) in die Cellulite-Haut gesichert werden.

### c) Darstellung der Erfindung

Diese Aufgabe wird erfindungsgemäß gelöst mit einer tagesrhythmisch angepassten Anwendung des Produktes Hyaferm in Kombination mit geeigneten Wirkstoffen.

### d) Vorteilhafte Wirkung der Erfindung

Das Konzept einer tagesrhythmisch angepassten Anwendung von Hyaferm^{®} in Kombination mit geeigneten Wirkstoffen bei Cellulite erscheint sinnvoll und sollte ausgehend von neuesten Ergebnissen der Chronobiologie, der Hormon-, Haut-, Fettgewebe-, Biochemie-, Physiologie- und Immunforschung realisiert werden.

Die genetische Anlage der zellulären Zeitgeber und die subtil verkoppelten Regulationssysteme der Periodizitäten Tag/Nacht und Füllung/Entleerung der Fettgewebe sind außerordentlich stabil und geben dem Gefüge der signalgebenden Elemente einen hohen Widerstand gegen Auslenkungen. Trotzdem können Altem, Emährungs- und Lebensweise (Fasten, Alkoholexzess, Rauchen, Übergewicht, Schlafstörungen) und Erkrankungen (Diabetes, Krebs, Cushing'sche Krankheit) mittel- und langfristig zu Veränderungen der regulierenden Stellgrößen und der Reaktionslage und zu Instabilität führen.

Anhand der zirkadianen Rhythmik von Leptin, Insulin, Cortisol, Lipoproteinlipase und der Körpertemperatur kann von einer Phase des Fetteinbaus in das Unterhautfettgewebe in der Nacht und der Mobilisation gespeicherten Fettes am Tage ausgegangen werden. Durch Auftragung eines geeigneten(r) Wirkstoffe(s) soH die Tages-Nacht-Rhythmik der Cellulite-Haut auf zweifache Weise beeinflusst werden:
• Schwächung des Fetteinbaus in der Nachtphase
• Förderung der Fettmobilisation in der Tagphase.
   Durch den Hyaferm^{®}-Zusatz soll die Permeation der/des Wirkstoffe(s) in die Cellulite-Haut gesichert werden.

### e) Literatur

Arasaradnam MP, Morgan L, Wright J, Gama R (2002) Diurnal variation in lipoprotein lipase activity. Ann. Clin. Biochem. 39:136-139.
Bergo M, Olivecrona G, Olivecrona T (1996) Diurnal rhythms and effects of fasting and refeeding an rat adipose tissue lipoprotein lipase. Am. J. Physiol. 271(6 Pt 1):E1092-E1097.
Bjorntorp P (1996) The regulation of adipose tissue distribution in humans. Int. J. Obes. Relat. Metab. Disord. 20:299-302.
Bjorntorp P (1997) Hormonal control of regional fat distribution. Hum. Reprod. 12(Suppl.1):21-25.
Bray MS, Young ME (2007) Circadian rhythms in the development of obesity: potential role for the circadian clock within the adipocytes. Obes. Rev. 8:169-181.
Ceddia RB, William WN Jr, Lima FB, Flandin P, Curi R, Giacobino JP (2000) Leptin stimulates uncoupling protein-2 mRNA expression and Krebs cycle activity and inhibits lipid synthesis in isolated rat white adipocytes. Eur. J. Biochem. 267:5952-5958.
Elimam A, Knutsson U, Bronnegard M, Stiema P, Albertsson-Wikland K, Marcus C (1998) Variations in glucocorticoid levels within the physiological range affect plasma leptin levels. Eur. J. Endocrinol. 139:615-620.
Franceschini R, Corsini G, Castaldi A, Fiorucci A, Tenerelli P, Rolandi E, Barreca T (1999) Twenty-four-hour variation in serum leptin in the elderly. Metabolism 48:1011-1014.
Gasquet de P, Griglio S, Pequignot-Planche E, Malewiak MI (1977) Diurnal changes in plasma and liver lipids and lipoprotein lipase activity in heart and adipose tissue in rats fed a high and low fat diet. J. Nutr. 107:199-212.
Hassager C, Ristelli J, Ristelli L, Jensen SB, Christiansen C (1992) Diurnal variation in serum markers of type I collagen synthesis and degradation in healthy premenopausal women. J. Bone Miner. Res. 7:1307-1311.
Havel PJ, Kasim-Karakas S, Mueller W, Johnson PR, Gingerich RL, Stern JS (1996) Relationship of plasma leptin to plasma insulin and adiposity in normal weight and overweight women: effects of dietary fat content and sustained weight loss. J. Clin. Endocrinol. Metab. 81:4406-4413.
Hayashi M, Shimba S, Tezuka M (2007) Characterization of the molecular clock in mouse peritoneal macrophages. Biol, Pharm. Bull. 30:621-626.
Ishida N (2007) Circadian clock, cancer and lipid metabolism. Neurosci. Res. 57:483-490.
Jackson BF, Blumsohn A, Goodship AE, Wilson AM, Price JS (2003) Circadian variation in biochemical markers of bone cell activity and insulin-like growth factor-I in two-year-old horses. J. Anim. Sci. 81:2804-2810.
Kondratov RV, Kondratova AA, Gorbacheva VY, Vykhovanets OV, Antoch MP (2006) Early aging and age-related pathologies in mice deficient in BMAL1, the core component of the circadian clock. Genes Dev. 20:1868-1873.
Larsson H, Ahren B (1996) Short-term dexamethasone treatment increases plasma leptin independently of changes in insulin sensitivity in healthy women. J. Clin. Endocrinol. Metab. 81:4428-4432.
Matkovic V, Ilich JZ, Badenhop NE, Skugor M, Clairmont A, Klisovic D, Landoll JD (1997) J. Clin. Endocrinol. Metab. 82:1368-1372.
McCarthy MF (2001) Modulation of adipocytes lipoprotein lipase expression as a strategy for preventing or treating visceral obesity. Med. Hypotheses 57:192-200.
Meyer W, Schönnagel B, Fleischer L-G (2006) A note on the integumental (1→3(1→6)ß-D-glucan permeation, using the porcine ear skin model. J. Cosmet. Dermatol. 5:130-134.
Miell JP, Englaro P, Blum WF (1996) Dexamethasone induces an acute and sustained rise in circulating leptin levels in normal human subjects. Horm. Metab. Res. 28:704-707.
Mirrashed F, Sharp JC, Krause V, Morgan J, Tomanek B (2004) Pilot study of dermal and subcutaneous fat structures by MRI in individuals who differ in gender, BMI, and cellulite grading. Skin Res. Technol. 10:161-168.
Rasmussen DD, Mitton DR, Larsen SA, Yellon SM (2001) Agingdependent changes in the effect of daily melatonin supplementation on rat metabolic and behavioural responses. J. Pineal Res. 31:89-94,
Reppert SM, Weaver DR (2001) Molecular analysis of mammalian circadian rhythms. Ann. Rev. Physiol. 63:647-676.
Saad ME, Riad-Gabriel MG, Khan A, Sharma A, Michael R, Jinagouda SD, Bovadjian R, Steil GM (1998) J. Clin. Endocrinol. Metab. 83:453-459. Nutritional regulation of leptin in humans. Diabetologia 42:639-646.
Shimba S, Ishii N, Ohta Y, Ohno T, Watabe Y, Hayashi M, Wada T, Aoyagi T, Tezuga M (2005) Brain and muscle Amt-like protein-1 (BMAL1), a component of the molecular clock, regulates adipogenesis. Proc. Natl. Acad. Sci. USA 102:12071-92076.
Simon C, Gronfier C, Schlienger JL, Brandenberger G (1998) Circadian and ultradian variations of leptin in normal man under continuous enteral nutrition: relationship to sleep and body temperature. J. Clin. Endocrinol. Metab. 83:1893-1899.
Strubbe JH, van Dijk G (2002) The temporal organization of ingestive behaviour and its interaction with regulation of energy balance. Neurosci. Biobeh. Rev. 26:485-498.
Vanitallie TB (2006) Sleep and energy balance: interactive homeostatic systems. Metabolism 55:(10; Suppl, 2):S30-S35).
Wabitsch M, Jensen PB, Blum WF, Christoffersen CT, Englaro P, Heinze E, Rascher W, Teller W, Tornquist H, Hauner H (1996) Insulin and cortisol promote leptin production in cultured human fat cells. Diabetes 45:1435-1438.
Wolthers CD, Heuck C, Skjaerbaek C (1999) Diurnal rhythm in serum leptin. J. Pediatr. Endocrinol. Metab. 12:863-866
Wu MS, Ho LT, Jap TS, Chen JJ, Kwok CF (1986) Diurnal variation of insulin clearance and sensitivity in normal man. Proc. Natl. Sei. Counc. Repub. China B 10:64-69.
Zhou YT, Shimabukuro M, Koyama K, Lee Y, Wang MY, Trieu F, Newgard CB, Unger RH (1997) Induction by leptin of uncoupling protein-2 and enzymes of fatty acid oxidation. Proc. Natl, Acad. Sci. 94:6386-6390.
Zvonic S, Ptitsyn AA, Conrad SA, Scott LK, Floyd ZE, Kilroy G, Wu X, Goh BC, Mynatt RL, Gimble JM (2006) Charaeterization of peripheral circadian clocks in adipose tissues. Diabetes 55:962-970.

## Patentansprüche

1. Verfahren der tagesrhythmisch angepassten Anwendung von Wirkstoffen bei Cellulite.

2. Zusatz von Hyaferm zu der Formulierung, die dem Auftragen der tagesrhytmisch angepassten Anwendung von Wirkstoffen bei Cellulite auf die Haut dient, zur Sicherung der effizienten Permeation der/des Wirkstoffes(s) in die Cellulite-Haut.

## Geänderte Patentansprüche

### Geänderte Patentansprüche gemäss Regel 137(2) EPÜ.

**1.** Zusammensetzungen zur Bekämpfung der Zellulitis, **dadurch gekennzeichnet, dass** sie aus Hefepilzen extrahiertes Glucaferm^{®} zusammen mit einem Hyaferm^{®} aus Bakterien enthalten

**2.** Zusammensetzungen gemäß Anspruch 1, **dadurch gekennzeichnet, dass** sie bis 3 Gew.-% Glucaferm^{®} und bis 5 Gew.-% Hyaferm^{®} enthalten

**3.** Zusammensetzungen gemäß Anspruch 1 und 2, **dadurch gekennzeichnet, dass** das Glucaferm^{®}, dass sie enthalten, aus Zellwänden der Hefe Saccharomyces cerevisiae hergestellt wurde

**4.** Zusammensetzungen gemäß Anspruch 1 bis 3, **dadurch gekennzeichnet, dass** die Zellwände der Hefe Saccharomyces cerevisiae autolytisch aufgeschlossen und schonend aufbereitet werden, das Glucaferm^{®} eine Molmasse von 80 bis 130 kD besitzt.

**5.** Zusammensetzungen gemäß Anspruch 1 und 2, **dadurch gekennzeichnet, dass** das Hyaferm^{®}, dass sie enthalten, aus Bakterienkulturen gewonnen wird.

**6.** Zusammensetzungen gemäß Anspruch 1, 2 und 5, **dadurch gekennzeichnet, dass** das Hyaferm^{®} aus Kulturen von Streptococcus spp., zweckmäßigerweise durch Mitglieder der Serologischen Gruppe C, insbesondere Streptococcus zooepidemicus oder S. equi hergestellt wird und das Produkt einen Hyaferm^{®}-Gehalt bis zu 5 Prozent besitzt.

**7.** Zusammensetzungen gemäß Anspruch 1 bis 6, **dadurch gekennzeichnet, dass** Glucaferm^{®} und Hyaferm^{®} in einem molekularen Komplex vorliegen.

**8.** Zusammensetzung der für die Anwendung am Tage vorgesehenen Mischung gemäß Anspruch 1 bis 7, **dadurch gekennzeichnet, dass** zusätzlich den Fettabbau fördernde Wirkstoffe wie Koffein aus Kaffee, Tee oder Guarana, Carnitin, Kreatin, Phloridzin oder Extrakte aus Bitterorangenblüten und Ginkgo biloba eingemischt werden

**9.** Zusammensetzung der für die Anwendung am Abend vorgesehenen Mischung gemäß Anspruch 1 bis 7, **dadurch gekennzeichnet, dass** zusätzlich den Fetteinbau hemmende Wirkstoffe wie Escin aus der Rosskastanie, Linolensäure, Rutin oder Extrakte aus Yamswurzel oder Hibiscusblüten eingemischt werden

**10.** Zusammensetzung der für die Anwendung am Tage vorgesehenen Mischung gemäß Anspruch 1 bis 8, **dadurch gekennzeichnet, dass** sie in Form einer Creme, einer Lotion, als Gel oder in aufgeschäumter Form vorliegt

**11.** Zusammensetzung der für die Anwendung am Abend vorgesehenen Mischung gemäß Anspruch 1 bis 7 und 9, **dadurch gekennzeichnet, dass** sie in Form einer Creme, einer Lotion, als Gel oder in aufgeschäumter Form vorliegt

**12.** verfahren zur Behandlung von Zellulitis, **dadurch gekennzeichnet, dass** man einmalig oder mehrfach tagsüber auf die Zellulitis -Haut eine geeignete Menge gemäß der Ansprüche 1 bis 8 und 10 aufträgt und einreibt.

**13.** verfahren zur Behandlung von Cellulite, **dadurch gekennzeichnet, dass** man einmalig oder mehrfach tagsüber auf die Zellulitis-Haut eine geeignete Menge gemäß der Ansprüche 1 bis 7, 9 und 11 aufträgt und einreibt.
